# EUROPÄISCHE PATENTSCHRIFT

(11) **EP 2 041 080 B1**
(45) Veröffentlichungstag und Bekanntmachung des Hinweises auf die Patenterteilung: **30.09.2015**
(21) Anmeldenummer: 07765403.6
(22) Anmeldetag: 13.06.2007
(51) Int. Cl.: C07C 319/08, C07C 321/04

(54) **VERFAHREN ZUR HERSTELLUNG VON ALKYLMERCAPTANEN IN EINEM MEHRZONENFESTBETTREAKTOR**
METHOD FOR PRODUCING ALKYLMERCAPTANS IN A MULTI-ZONE FIXED-BED REACTOR
PROCÉDÉ DE FABRICATION D'ALKYLMERCAPTANS DANS UN RÉACTEUR À LIT FIXE À PLUSIEURS ZONES

(30) Priorität: 13.07.2006 DE 102006032635
(43) Veröffentlichungstag der Anmeldung: 01.04.2009
(73) Patentinhaber: Evonik Degussa GmbH, 45128 Essen (DE)
(72) Erfinder: REDLINGSHÖFER, Hubert, 91481 Münchsteinach (DE); WECKBECKER, Christoph, 63584 Gründau-Lieblos (DE)
(86) Internationale Anmeldenummer: PCT/EP2007/055852
(87) Internationale Veröffentlichungsnummer: WO 2008/006657

(56) Entgegenhaltungen:
- WO-A-2005/021491
- WO-A-2006/063669

## Beschreibung

Die Erfindung betrifft ein Verfahren zur Herstellung von Alkylmercaptanen durch katalytische Gasphasenumsetzung von Alkanolen und Schwefelwasserstoff an Alkaliwolframaten, wobei die Umsetzung in mindestens zwei aufeinanderfolgenden Reaktionszonen durchgeführt wird, die Katalysatoren unterschiedlicher Aktivität und Selektivität enthalten.

Unter den Alkylmercaptanen ist insbesondere Methylmercaptan ein industriell wichtiges Zwischenprodukt zum Beispiel für die Synthese von Methionin sowie für die Synthese von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drücken zwischen 1 und 25 bar.

Das Reaktionsgemisch enthält neben dem gebildeten Methylmercaptan die nicht umgesetzten Ausgangsstoffe und Nebenprodukte, wie zum Beispiel Dimethylsulfid und Dimethylether sowie Gase, wie zum Beispiel Methan, Kohlenmonoxid, Wasserstoff und Stickstoff. Aus diesem Reaktionsgemisch wird das gebildete Methylmercaptan abgetrennt.

Für die Wirtschaftlichkeit des Verfahrens wird eine möglichst hohe Ausbeute bei der katalytischen Reaktion von Methanol und Schwefelwasserstoff zu Methylmercaptan gefordert, um den Aufwand bei der Abtrennung des gebildeten Methylmercaptans vom Reaktionsgemisch so gering wie möglich zu halten. Hier stellt insbesondere der Energieaufwand zur Kühlung des Reaktionsgasgemisches zur Kondensation des Methylmercaptans einen großen Kostenfaktor dar.

Zur Erhöhung von Aktivität und Selektivität wird Aluminiumoxid als Träger üblicherweise mit Kaliumwolframat oder Cäsiumwolframat versetzt. Dabei wird das Wolframat gewöhnlich in Mengen bis 25 Gew.-%, bezogen auf das Gesamtgewicht des Katalysators, eingesetzt. Eine Verbesserung von Aktivität und Selektivität erhält man auch durch Erhöhung des Molverhältnisses von Schwefelwasserstoff zu Methanol. Üblicherweise werden Molverhältnisse zwischen 1 und 10 angewendet.

Ein hohes Molverhältnis bedeutet allerdings auch einen hohen Überschuss des Schwefelwasserstoffes im Reaktionsgemisch und somit die Notwendigkeit, große Gasmengen im Kreis zu führen. Zur Verminderung des hierfür benötigten Energieaufwandes sollte daher das Verhältnis von Schwefelwasserstoff zu Methanol nur wenig von 1 abweichen.

In der EP 0 832 687 B1 werden die Vorteile der Verwendung von Cäsiumwolframat (Cs₂WO₄) anstelle von Kaliumwolframat (K₂WO₄) als Promotor beschrieben. So kann durch Einsatz von Cäsiumwolframat eine gesteigerte Aktivität bei gleichzeitig guter Selektivität erreicht werden.

Durch Erhöhung der Cäsiumwolframat-Konzentration auf bis zu 40 Gew.-% kann die Selektivität zu Methylmercaptan auf bis zu 92 % gesteigert werden, ohne dass die Aktivität unverhältnismäßig verschlechtert wird.

Nach allgemeiner Ansicht erzielt man die beste Selektivität mit Katalysatoren, bei denen das Alkali/Wolframverhältnis gleich 2:1 ist (A.V. Mashkina et al., React. Kinet. Catal. Lett., Vol. 36, No. 1, 159-164 (1988).

Diese Untersuchungen berücksichtigen nicht, dass die Konzentrationsverhältnisse von Reaktanten und Produkten, aber auch Temperaturen im Reaktor sich im Verlauf der Reaktion stark unterscheiden.

Aufgabe der vorliegenden Erfindung ist es, ein Verfahren bereitzustellen, das sich durch eine verbesserte Ausbeute gegenüber den bekannten Verfahren auszeichnet und somit zu einer höheren Wirtschaftlichkeit führt.

Gegenstand der Erfindung ist ein Verfahren zur Herstellung von Alkylmercaptanen, insbesondere Methylmercaptan, durch katalytische Gasphasenumsetzung von Alkanolen, insbesondere Methanol und Schwefelwasserstoff, an einem festen Alkaliwolframat, wobei die Umsetzung der Reaktanten in mindestens zwei aufeinanderfolgenden Reaktionszonen erfolgt, die unterschiedliche Katalysatoren mit an die Konzentrationsverhältnisse der Reaktanten in diesen Zonen in Hinblick auf die Mercaptanbildung angepassten Aktivitäten enthalten, wobei die Aktivität des in der ersten Reaktionszone eingesetzten Katalysators bezogen auf die Alkylmercaptanbildung niedriger ist als die des in der zweiten oder weiteren Reaktionszone(n) eingesetzten Katalysators.

Durch diese Abstufung wird die Tatsache berücksichtigt, dass die Konzentration der Edukte während des Durchströmens der Reaktionszonen abnimmt, während gleichzeitig die Konzentration des Zielprodukts ansteigt. Weiterhin wird berücksichtigt, dass sich bei der hier vorliegenden stark exothermen Reaktion eine erhebliche Übertemperatur vor allem im ersten Teil des Festbetts einstellen kann, da dort die Konzentrationen der Edukte am höchsten sind.

Die Einrichtung von Reaktionszonen mit unterschiedlicher Aktivität ermöglicht auch die Steigerung der Anlagenlast bzw. der Raum-Zeit-Ausbeute, da durch die nicht vollständige Umsetzung am weniger aktiven Katalysator in der ersten Zone dort eine geringere Exotherme (maximale Übertemperatur im Festbett) erzeugt wird. Die frei werdende Wärmemenge verteilt sich erfindungsgemäß gleichmäßiger über den Reaktor. Dies ermöglicht wiederum eine Anhebung des Eduktstromes, der durch eine maximal zulässige Exotherme begrenzt ist.

Diese Vorteile beruhen auf der erfindungsgemäßen Maßnahme, Katalysatoren mit unterschiedlichen Aktivitäten, Selektivitäten oder auch thermischer Belastbarkeit, in den verschiedenen Zonen zu verwenden. In der zweiten oder weiteren folgenden Reaktionszonen wird bevorzugt ein Katalysator mit einer höheren Aktivität für die Alkylmercaptanbildung als in der ersten Reaktionszone eingesetzt, der geeignet ist, auch bei der geringen Konzentration der Reaktanten im zweiten Reaktionsraum die Alkylmercaptanbildung zu beschleunigen und zu vervollständigen. Gleichzeitig katalysiert er bevorzugt nicht oder nur unwesentlich (Selektivität bevorzugt ≤ 1%) die Nebenproduktbildung aus dem dort im Vergleich zu den Edukten im Überschuß vorhandenen Alkylmercaptan z. B. zum Dimethylsulfid oder Dimethyldisulfid.

In einer Ausführungsform des erfindungsgemäßen Verfahrens katalysiert daher der Katalysator in der zweiten Zone die Umsetzung des entstandenen Alkylmercaptans zu Nebenprodukten mit einer Selektivität von ≤ 1%.

In der ersten Reaktionszone setzt man dagegen bevorzugt einen Katalysator ein, der zusätzlich zur Alkylmercaptanbildung die Umsetzung von Alkanolen und Schwefelwasserstoff in parallel verlaufenden Umsetzungen z. B. zum Dimethylether nicht oder nur unwesentlich (Selektivität bevorzugt ≤ 1 %) katalysiert. Diese Katalysatoren sind bevorzugt in Hinblick auf die Mercaptanbildung weniger aktiv als die Katalysatoren der zweiten Reaktionszone. Weiterhin sind die Katalysatoren für die erste Reaktionszone bevorzugt thermisch stärker belastbar und liefern auch bei hohen Temperaturen mit guter Selektivität Alkylmercaptan.

In einer Ausführungsform des erfindungsgemäßen Verfahrens katalysiert daher der Katalysator in der ersten Zone die Umsetzung von Alkanolen und Schwefelwasserstoff zu Nebenprodukten mit einer Selektivität von ≤ 1%.

Zwischen den Reaktionszonen erfolgt keine Einspeisung von Reaktanten. Die Aktivitäten und Selektivitäten der einzusetzenden Katalysatoren sind durch Standardversuche leicht zu ermitteln. Die Aktivität kann in mol Mercaptan /(m³ Reaktionsraum * h) oder % Umsatz Alkanol angegeben werden. Die Aktivität des Katalysators ist in der zweiten Reaktionszone unter gleichen Standardversuchsbedingungen um mindestens 1 % höher als die des in der ersten Reaktionszone eingesetzten, insbesondere um 10 bis 50% höher.

Bevorzugt sind Reaktionszonen in Form von Festbettschüttungen, die direkt miteinander verbunden sind. Die erfindungsgemäße Verknüpfung von Reaktionszonen ist unabhängig von der Ausführung des Reaktors. So können in allen technischen Festbettreaktoren mehrere Reaktionszonen implementiert werden. Zum Beispiel würden in einem senkrechten Rohrreaktor in Strömungsrichtung unterschiedliche Reaktionszonen durch Befüllung des Rohres mit verschiedenen Katalysatoren auf unterschiedlicher Höhe entstehen. Die Reaktionszonen können zusätzlich durch unterschiedliche Geometrien oder durch separate Heiz- oder Kühlkreisläufe von Wärmeträgermedien optimiert sein.

Die Länge, der Durchmesser und das Volumen der einzelnen Reaktionszonen hängt von der Aktivität der eingesetzten Katalysatoren ab.

In der 1. Zone setzt man bevorzugt einen Katalysator mit einer Aktivität bei der Mercaptanbildung ein, die geringer ist als die des in dem oder den folgenden Abschnitten eingesetzten.

Auf diese Weise vermeidet man bei der vorliegenden exothermen Reaktion sehr hohe lokale Überhitzungen im 1. Abschnitt während die höhere Aktivität für die Mercaptanbildung im zweiten Abschnitt zu einem nahezu vollständigen Umsatz der noch nicht umgesetzten Reaktanten führt. Für Methanol wurde erfindungsgemäß ein Umsatzgrad von 99% gemessen.

Der Wechsel zu einem Katalysator mit höherer Aktivität findet im allgemeinen nach Umsetzung von 30 bis 95 Mol%, bevorzugt 50 bis 90, insbesondere 60 bis 90 % des Alkohols zum Alkylmercaptan statt.

In einer Ausführungsform des erfindungsgemäßen Verfahrens erfolgt daher der Wechsel zu einem Katalysator mit einer gegenüber dem in der ersten Reaktionszone eingesetzten höheren Aktivität für die Mercaptanbildung nach der Umsetzung von 30 bis 95% des eingesetzten Alkohols in der ersten Reaktionszone.

Als Katalysatoren dienen insbesondere halogenidhaltige und halogenidfreie Alkaliwolframate oder Kombinationen dieser Verbindungen.

In einer Ausführungsform des erfindungsgemäßen Verfahrens setzt man daher halogenidhaltige und/oder halgenidfreie Alkaliwolframate als Katalysatoren ein.

Diese sind bekannt aus den Anmeldungen WO 2005/021491, WO 2004/096760, WO 2006/015668 und aus der PCT/EP/2005/012598.

Dabei werden halogenidhaltige, insbesondere Bromverbindungen enthaltende Katalysatoren, bevorzugt im 2. Abschnitt eingesetzt.

In einer weiteren Ausführungsform des erfindungsgemäßen Verfahrens setzt man in der ersten Reaktionszone ein halogenidfreies und in der zweiten Reaktionszone ein halogenidhaltiges Alkaliwolframat ein.

In einer bevorzugten Ausführungsform des erfindungsgemäßen Verfahrens setzt man ein bromidhaltiges Alkaliwolframat ein.

Die erfindungsgemäß eingesetzten bevorzugt halogenidhaltigen Katalysatoren haben im allgemeinen die Formel

A_{X}WO_{Y}X_{Z},

in der bedeuten,
A: mindestens ein Alkalimetall, insbesondere ausgewählt aus der Gruppe Na, K, Cs, Rb;
X: mindestens ein Halogenid, ausgewählt aus der Gruppe F, Cl, Br, J
x: 0,1 bis 4, insbesondere 1,2 bis 3;
Y: dieser Wert stellt sich entsprechend der Struktur des Wolframats und des Alkaligehalts aufgrund der 6-Wertigkeit des Wolframs ein;
Z: 0,01 bis 12, insbesondere 0,9 bis 4.

Die Größe von z ist ein Maß für den Halogenidgehalt im Wolframat, der nicht chemisch an das Wolframat gebunden vorliegen muss.

Der Halogenidbestandteil der Zusammensetzung gemäss Formel I besteht aus oder enthält insbesondere dann Chlorid, wenn das Wolframat mindestens zwei verschiedene, gebundene Alkalimetalle und/oder mindestens ein weiteres Halogenid, ausgewählt aus der Gruppe F, Br, J, enthält.

Chlorid ist bevorzugt allein als Halogenid enthalten, wenn das molare Verhältnis von Na oder K/W im Katalysator >0,9 bis 1,9 ist.

Der Alkalibestandteil der katalytisch aktiven Verbindung kann sich aus einem oder mehreren Elementen der Alkaligruppe zusammensetzen. Der gebundene Halogenbestandteil des Katalysators ebenso kann sich aus einem oder mehreren verschiedenen Halogeniden zusammensetzen.

Liegt der Katalysator als Trägerkatalysator vor, enthält er das halogenidhaltige Alkaliwolframat in einer Menge von 8 bis 50 Gew.-%, insbesondere 15 bis 40 Gew.-%, bevorzugt 20 bis 36 Gew.-%. Im Falle eines Schalenkatalysators beziehen sich diese Anteile auf die Zusammensetzung der Schale.

Die halogenhaltigen oxidischen Verbindungen aus Alkalimetall(en) und Wolfram können direkt auf einen Trägerkörper imprägniert werden (Trägerkatalysator).

Bei der Herstellung von Katalysatoren in Form von Extrudaten oder Preßlingen wird der pulverförmige Träger mit der erfindungsgemäßen oxidischen Zusammensetzung imprägniert oder vermischt und die erhaltene Zwischenstufe anschließend verformt (Vollkatalysator). Wird ein Schalenkatalysator hergestellt, so wird der pulverförmigen Träger mit der katalytisch wirksamen Zusammensetzung imprägniert und die entstehende Mischung dann auf einen bevorzugt inerten Trägerkern in Form einer Schale aufgebracht.

Das molare Halogenid/Alkali-Verhältnis beläuft sich besonders bevorzugt auf 0,1:1 bis 1:1. Damit enthalten die erfindungsgemäßen Wolframate für die Umsetzung von Alkanolen mit Schwefelwasserstoff zu Alkylmercaptanen im Unterschied zu den mit Cäsiumwolframat (Cs₂WO₄) oder Kaliumwolframat (K₂WO₄) imprägnierten Katalysatoren gemäss Stand der Technik einen Anteil an Halogeniden.

Es zeigt sich, dass der Anteil an Halogeniden insbesondere auf dem bevorzugt eingesetzten Aluminiumoxid im Vergleich zu dem im Stand der Technik ausschließlich verwendeten nicht halogenidfreien Alkaliwolframat dem Katalysator eine deutlich verbesserte Aktivität bei gleichzeitig hoher Selektivität verleiht. Ferner zeigt sich durch den Zusatz von Halogeniden zum Alkaliwolframat in unerwarteter Weise eine ausgezeichnete Selektivität bei sehr hohen Umsatzgraden an Alkohol. Erfindungsgemäß kann bei sehr hohen Beladungen mit dem Promotor ein ausgezeichneter Umsatz erreicht werden, ohne dass die Selektivität des Katalysators abnimmt, wie es aus dem Stand der Technik für halogenidfreien Katalysatoren bekannt ist. Weiterhin wurde gefunden, dass über das Alkali-Wolfram-Halogenid-Verhältnis und über die Wahl der Alkalimetalle und der Halogenide die Aktivität und Selektivität des Katalysators gezielt eingestellt werden kann. Durch die Möglichkeit, Mischungen von Verbindungen verschiedener Alkalimetalle oder Halogene einzusetzen, können vergleichsweise teure Substanzen wie Cäsium-, Rubidium-, Brom- oder Jod-Verbindungen zumindest teilweise durch kostengünstigere wie z.B. Kalium oder Natrium-Verbindungen oder Chloride ersetzt werden, ohne dass die Aktivität oder Selektivität des Katalysators beeinträchtigt wird.

Die erfindungsgemäß verwendeten Katalysatoren werden bevorzugt in Form eines Trägerkatalysators, bei dem die Oberfläche mit der katalytisch wirksamen Substanz imprägniert wird, oder eines Schalenkatalysators, bei dem ein bevorzugt inerter Kern mit einem Gemisch aus katalytisch aktiver Substanz und Trägermaterial umgeben ist, eingesetzt. Weiterhin können Extrudate oder Preßlinge, bei denen die katalytisch aktive Substanz mit dem pulverförmigen Trägermaterial vor der Verformung vermischt bzw. diese mit ihr imprägniert wird, verwendet werden.

Als Trägermaterialien werden die bekannten oxidischen anorganischen Verbindungen wie zum Beispiel SiO₂, TiO₂, ZrO₂ und bevorzugt sogenanntes aktives Aluminiumoxid ausgewählt.

Dieses Material weist hohe spezifische Oberflächen zwischen etwa 10 und 400 m²/g auf und besteht hauptsächlich aus Oxiden der Übergangsreihe der kristallographischen Phasen des Aluminiumoxids (siehe zum Beispiel Ullmann's Encyclopedia of Industrial Chemistry von 1985, Vol. A1, Seiten 561-562). Zu diesen Übergangsoxiden gehören γ-, δ-, η-, κ-, χ- und θ-Aluminiumoxid. Alle diese kristallographischen Phasen gehen bei Erhitzung des Aluminiumoxids auf Temperaturen über 1100°C in das thermisch stabile α-Aluminiumoxid über. Aktives Aluminiumoxid wird kommerziell für katalytische Anwendungen in verschiedenen Qualitäten und Lieferformen angeboten. Besonders geeignet für die Herstellung von Trägerkatalysatoren sind Formkörper aus granuliertem oder stranggepresstem Aluminiumoxid mit Korndurchmessern von 1 bis 5 mm, einer spezifischen Oberfläche von 180 bis 400 m²/g, einem Gesamtporenvolumen zwischen 0,3 und 1,2 ml/g sowie einer Schüttdichte von 300 bis 900 g/l. Für die Zwecke der Erfindung wird bevorzugt Aluminiumoxid mit mehr als 200 m²/g spezifischer Oberfläche verwendet, da die katalytische Aktivität des fertigen Katalysators mit steigender Oberfläche des Aluminiumoxids leicht ansteigt. Dieses Material wird in Pulverform bevorzugt für die Herstellung der Schalenkatalysatoren, Extrudate oder Preßlinge eingesetzt.

Die wässrige Imprägnierlösung zur Aufbringung des Promotors kann in einfacher Weise aus in Wasser löslichen Alkali-, Wolfram- und Halogenverbindungen, insbesondere Wolframsäure (H₂WO₄), Alkalihydroxiden, gegebenenfalls Alkalihalogeniden oder Ammoniumhalogenide oder Halogenwasserstoffsäure hergestellt werden. Hierzu wird z. B. Wolframsäure in Wasser suspendiert und unter Zugabe einer Base und Erwärmen aufgelöst. Das oder die gewünschten Alkalihalogenid(e)oder Ammoniumhalogenide, gegebenenfalls auch die entsprechenden Hydroxide und/oder beispielsweise gegebenenfalls eine Halogenwasserstoffsäure mit dem gegebenenfalls gewünschten Halogenid werden ebenfalls in Wasser aufgelöst und mit der Lösung der Wolframsäure so vereinigt (Promotorlösung), dass sich die gewünschte Zusammensetzungsverhältnisse für die Alkaliwolframate und ihren Halogenidgehalt ergeben. Vorteilhaft einsetzbar sind neben den Alkalihalogeniden auch Alkalisalze, deren Anionen sich durch Wärmebehandlung rückstandslos austreiben lassen wie z.B. Nitrate, Formiate, Oxalate, Acetate oder Carbonate. Zur Stabilisierung der Promotorlösung mit einem pH von bevorzugt 8 bis 14 werden anorganische und auch organische Basen eingesetzt. Bevorzugt werden solche verwendet, die sich durch eine abschließende Wärmebehandlung des nach der Imprägnierung erhaltenen Katalysators rückstandslos austreiben lassen. Zu diesen Basen gehören bevorzugt Ammoniumhydroxid und organische Basen, insbesondere Amine.

Durch dieses Vorgehen werden die an der Oberfläche von zum Beispiel Al₂O₃-Trägermaterialien vorhandenen sauren Gruppen weitgehend, im allgemeinen mindestens etwa 75 %, im besonderen 100 % neutralisiert.

Das molare Verhältnis von Alkalimetallverbindungen und Halogeniden in der wässrigen Imprägnierlösung wird derart gewählt, dass die neuen Wolframate Halogenide und Alkalimetalle in einem molaren Verhältnis von 0,01:1 bis 3:1 enthalten. Dies führt im Vergleich zu den bekannten halogenidfreien Katalysatoren zu einer deutlich erhöhten Ausbeute bei Verwendung der erfindungsgemäßen Katalysatoren, insbesondere bei niedrigen Verhältnissen von Schwefelwasserstoff und Methanol im Reaktionsgas. Bevorzugt sind Cäsium-, Kalium- und Rubidiumwolframate, insbesondere Cäsiumwolframate, als Halogenide werden Fluorid, Bromid und Chlorid bevorzugt, insbesondere Fluorid und Bromid.

Wolframate mit unterschiedlichen Alkalikationen oder Gehalten von verschiedenen Halogeniden enthalten bevorzugt Kationen von zwei verschiedenen Alkalimetallen und mindestens einem Halogenid bevorzugt in einem Verhältnis von Halogenid zu Alkali zwischen 0,01:1,0 und 3,0:1,0, wobei die molaren Anteile von Alkalimetallen bzw. gegebenenfalls vorhandenen unterschiedlichen Halogeniden als Summe gezählt werden. Dabei wird der Anteil des kostengünstigeren Alkalimetalls oder Halogenids so weit gesteigert und gleichzeitig derjenige des vergleichsweise teureren Alkalimetalls oder Halogenids im Gegenzug verringert, dass keine Verschlechterung der Aktivität oder Selektivität des Katalysators eintritt.

Bei Kombinationen von Alkalimetallen sind Wolframate, in denen der Cs- oder Rb-Anteil im vorteilhaften Verhältnis durch K- oder Na-Kationen ersetzt wird, bevorzugt.

Bevorzugt sind Katalysatoren, bei denen auch von einem molaren 1:1 Verhältnis abwechselnde Kombinationen von gebundenen Alkalimetallen aus der Gruppe
Kalium und Cäsium,
Natrium und Cäsium,
Rubidium und Cäsium,
Natrium und Kalium,
Rubidium und Kalium auftreten.

Allgemein können für das Aufbringen der Promotorlösung verschiedene Imprägniertechniken wie das Tauchimprägnieren, Sprühimprägnieren, Vakuumimprägnieren und die Porenvolumenimprägnierung eingesetzt werden, wobei die Imprägnierung auch mehrfach erfolgen kann. Bei Formkörpern muss das gewählte Imprägnierverfahren es ermöglichen, die gewünschte Beladungsmenge des Promotors mit guter Gleichmäßigkeit über den gesamten Querschnitt aufzubringen.

Bevorzugt wird die Promotorlösung durch Sprüh- oder Vakuumimprägnierung in einem oder in zwei Schritten auf die Formkörper aufgebracht. Bei der Sprühimprägnierung wird die wässrige Imprägnierlösung auf die Trägerkörper gesprüht. Bei der Vakuumimprägnierung wird in einem mit den Formkörpern gefüllten Behälter mittels einer Vakuumpumpe ein Unterdruck erzeugt. Durch Öffnen einer Verbindung zur wässrigen Imprägnierlösung wird die Lösung in den Behälter gesaugt, bis die gesamte Schüttung der Formkörper mit der Lösung bedeckt ist. Nach einer Imprägnierzeit von 0,2 bis 2 Stunden wird die nicht durch das Material aufgenommene Lösung abgelassen oder abgegossen.

Durch Vortrocknung für die Dauer von 1 bis 10 Stunden bei Raumtemperatur kann sich das anfängliche Konzentrationsgefälle über den Querschnitt der Formkörper weitgehend ausgleichen. Somit wird die Gleichmäßigkeit der Imprägnierung über den Querschnitt der Katalysatorpartikel verbessert. Bevorzugt werden die so erhaltenen Katalysatorvorstufen für die Dauer von 1 bis 10 Stunden bei 100 bis 200, bevorzugt 100 bis 140°C zur Entfernung der Restfeuchte getrocknet. Dann erfolgt für die Dauer von 1 bis 20, bevorzugt 1 bis 5 Stunden eine Kalzinierung bei 300 bis 600, bevorzugt 420 bis 480°C. Dadurch wird der Promotor auf dem Aluminiumoxid fixiert und die Base der Imprägnierlösung zersetzt und ausgetrieben. Optional kann die Schüttung der Trägerkörper der Katalysatorvorstufen bei der Vortrocknung, Trocknung und Kalzinierung von einem Gasstrom durchströmt werden, was den Abtransport der Restfeuchte und der Zersetzungsgase verbessert.

Die Imprägnierung der Formkörper kann auch mehrstufig, insbesondere zweistufig erfolgen.

In dieser bevorzugten Ausführungsform enthält dann die in der ersten Stufe eingesetzte Lösung ein bis zu zwei Drittel der vorgesehenen Gesamtmenge an Alkali- und Wolframverbindungen.

Geht man mehrstufig, mindestens aber zweistufig vor, kalziniert man die im ersten Schritt erhaltene Vorstufe gegebenenfalls nicht.

Ansonsten läuft in der zweiten Stufe dasselbe Imprägnier-, Trocknungs- und Kalzinierungsprogramm wie für das einstufige Verfahren beschrieben ab.

Diese mehrstufige Imprägnierung ist vor allem dann sinnvoll, wenn hohe Beladungen gewünscht werden und/oder die begrenzte Löslichkeit der Promotormischung die Beladung in einem Schritt nicht ermöglichen.

Es besteht auch die Möglichkeit, die Trägerkörper bzw. das Trägermaterial während des Imprägniervorgangs mehrfach mit der Imprägnierlösung zu besprühen und zwischen diesen Behandlungsschritten jeweils Teile der Restfeuchte bei einer Temperatur von bis zu 120°C zu entfernen.

Bei der Herstellung des Schalenkatalysators kann das zur Bildung einer Schale aufzubringende Pulver vor oder nach der Beschichtung kalziniert werden. Beispielsweise kann dieser Katalysatortyp gemäß EP-B-O 068 193 hergestellt werden. Auch bei der Herstellung der Extrudate oder der Preßlinge kann die Kalzinierung vor und/oder nach der Verformung erfolgen.

Außer durch die chemische Zusammensetzung der Katalysatoren kann die unterschiedliche Aktivität in den Reaktionszonen auch durch Veränderung der physikalischen Eigenschaften der Katalysatoren erreicht werden. Dem Fachmann ist bekannt, wie die physikalischen Eigenschaften eines Katalysators zu verändern sind, um die Aktivität zu erhöhen oder zu senken. Demzufolge können in den unterschiedlichen Reaktionszonen auch Katalysatoren mit gleicher chemischer Zusammensetzung aber unterschiedlicher spezifischer Oberfläche, Größe, Form (z.B. Kugel, Zylinder, Hohlzylinder), Porenradienverteilung und/oder unterschiedlichem Porenvolumen eingesetzt werden.

Unterschiedliche Aktivitäten im ersten und den folgenden Reaktionsräumen kann man auch durch Verdünnung der Katalysatormenge mit Intertmaterialien (z.B. durch porenfreie Formkörper wie Glaskugeln) erzeugen. In den darauffolgenden Reaktionszonen wird der Katalysator dann erfindungsgemäß mit weniger Inertmaterial verdünnt oder unverdünnt eingesetzt. Die Verdünnung wird z. B. durch eine Vormischung des Katalysators mit dem Intertmaterial (mechanische Mischung) erreicht, die anschließend in die Reaktionszone gefüllt wird. Das bevorzugte VolumenVerhältnis von Katalysator zu Inertmaterial beträgt dabei je nach Reaktionszone zwischen 1:0 und 1:2. Im Falle der Verdünnung des Katalysatormenge mit Inertmaterial wird in den verschiedenen Reaktionszonen bevorzugt der gleiche Katalysator eingesetzt.

In einer Ausführungsform des erfindungsgemäßen Verfahrens wird daher die geringere Aktivität des Katalysators in der ersten Reaktionszone durch die Verdünnung der Katalysatormenge mit festem Inertmaterial erreicht.

In einer bevorzugten Ausführungsform des erfindungsgemäßen Verfahrens wird in der ersten und den zweiten oder weiteren Reaktionszone(n) derselbe Katalysator eingesetzt.

In einer weiteren Ausführungsform des erfindungsgemäßen Verfahrens wird die geringere Aktivität des Katalysators in der ersten Reaktionszone durch die Variation von einer oder mehreren physikalischen Eigenschaften ausgewählt aus der Gruppe spezifische Oberfläche, Porenradienverteilung, Porenvolumen, Pelletdurchmesser und Pelletform erreicht. Diese Maßnahme kann man auch mit der oben beschriebenen Verwendung von Katalysatoren unterschiedlicher Zusammensetzung kombinieren.

In einer weiteren Ausführungsform der Erfindung sind die Reaktionszonen mit unterschiedlicher Aktivität nicht direkt miteinander verbunden, sondern befinden sich in separaten Apparaten, die über Rohrleitungen miteinander verbunden sind. Dies kann beispielsweise durch die Reihenschaltung von Reaktoren erreicht werden. Dabei können sich die Reaktoren in Bauart, Größe und Strömungsführung unterscheiden. Ein wesentlicher Vorteil bei der Aufteilung auf verschiedene Reaktoren besteht darin, dass die Reaktionsbedingungen in den unterschiedlichen Reaktionszonen einfacher unabhängig voneinander eingestellt werden können. Weiterhin können die Katalysatoren besser separat ausgetauscht werden.

In einer Ausführungsform des erfindungsgemäßen Verfahrens folgen daher die Reaktionszonen aufeinander in unterschiedlichen Reaktoren, die miteinander in Reihe geschaltet sind.

Das erfindungsgemäße bevorzugte Verfahren zur Herstellung von Methylmercaptanen durch Umsetzung von Schwefelwasserstoff und Methanol an einem Katalysator wird im allgemeinen als Gasphasenreaktion in einem Rohereaktor durchgeführt. Es können auch mehrere Rohrreaktoren hintereinander geschaltet verwendet werden. Im allgemeinen werden Methanol und Schwefelwasserstoff auf eine Temperatur geheizt, die hoch genug ist, dass sowohl Methanol als auch Methylmercaptan in der Dampfphase vorliegen, die jedoch unter der Zersetzungstemperatur des Methylmercaptans liegt. Im allgemeinen wird das erfindungsgemäße Verfahren bei Temperaturen zwischen 250 und 500°C, bevorzugt zwischen 300 und 450° durchgeführt. Die genaue Reaktionstemperatur ist unter anderem abhängig vom Reaktionsdruck und dem eingesetzten Katalysator.

In einer anderen Ausführungsform des erfindungsgemäßen Verfahrens sind die Reaktionszonen direkt miteinander verbunden.

In einer weiteren Ausführungsform des erfindungsgemäßen Verfahrens setzt man Schwefelwasserstoff und Methanol zu Methylmercaptan um.

Das erfindungsgemäße Verfahren wird im allgemeinen bei einem Druck von 1 bis 25 bar durchgeführt. Selbstverständlich wird der Druck nicht so hoch gewählt, dass der Reaktorfeed bzw. das Methylmercaptan kondensiert. Bevorzugt beträgt der Druck in dem erfindungsgemäßen Verfahren 1 bis 10 bar. Aus Gründen eines verminderten Emissionsrisikos kann er auf 1 bis 3 bar eingestellt werden, bevorzugt annähernd drucklos.

Das erfindungsgemäße Verfahren wird im allgemeinen kontinuierlich durchgeführt. Die Aufarbeitung des erhaltenen Methylmercaptans erfolgt dabei nach dem Fachmann bekannten Methoden.

Die WHSV (Weight hourly space velocity = Gewichte Edukte / Gewicht Katalysator pro Rohr und Stunde) beträgt im allgemeinen 0,1 bis 10 h⁻¹, bevorzugt 0,1 bis 5 h⁻¹, insbesonderes bevorzugt 0,5 bis 2h⁻¹.

Das erfindungsgemäße Verfahren besitzt den Vorteil, dass die eingesetzten Katalysatoren nicht im Hinblick auf alle Parameter hin optimiert werden müssen. Dies ist der Fall, wenn nur ein Katalysatortyp verwendet wird.

Dieser muß dann gleichzeitig eine hohe Aktivität hohe Selektivität für die Mercaptanbildung und möglichst niedrige Selektivitäten für die Bildung von Paralellprodukten aus den Eduktgasen bzw. Nebenprodukten aus der Umsetzung der Mercaptane miteinander aufweisen. Die dabei auftretenden Probleme führen dann im Gegensatz zum erfindungsgemäßen Verfahren immer zu Verlusten bei der Mercaptanbildung.

### Beispiele

### Vergleichsbeispiel 1:

In einem beheizten Rohrreaktor mit einem Reaktionsraum von 1,4 l wurde ein Eduktstrom von 0,5 kg/h Methanol und 0,95 kg/h Schwefelwasserstoff bei einem Druck von 9 bar eingespeist. Der Reaktionsraum wurde vollständig mit einem Katalysator gemäß WO 2005/021491 befüllt. Bei einer Wandtemperatur von 330 °C und einer Reaktionstemperatur zwischen 330 und 380 °C innerhalb des Reaktionsraumes stellte sich am Reaktorausgang ein Umsatzgrad an Methanol von 90 % ein. Die Selektivität zu Methylmercaptan betrug 96 %, die Ausbeute an Methylmercaptan 86,5 %.

### Vergleichsbeispiel 2:

In einem beheizten Rohrreaktor mit einem Reaktionsraum von 1,4 l wurde ein Eduktstrom von 0,5 kg/h Methanol und 0,95 kg/h Schwefelwasserstoff bei einem Druck von 9 bar eingespeist. Der Reaktionsraum wurde vollständig mit einem Katalysator gemäß PCT/EP/2005/012598 befüllt, wobei der Halogenidgehalt (Br) des Katalysators 9,5 Gew.-% betrug.

Bei einer Wandtemperatur von 320 °C und einer Reaktionstemperatur zwischen 320 und 370 °C innerhalb des Reaktionsraumes stellte sich am Reaktorausgang ein Umsatzgrad an Methanol von 99 % ein. Die Selektivität zu Methylmercaptan betrug 93 %, die Ausbeute an Methylmercaptan 92 %.

### Beispiel:

### In einem beheizten Rohrreaktor mit einem

Gesamtreaktionsraum von 1,4 l wurde ein Eduktstrom von 0,5 kg/h Methanol und 0,95 kg/h Schwefelwasserstoff bei einem Druck von 9 bar eingespeist. Der Reaktionsraum wurde in der ersten, oberen Reaktionszone (60 % des Gesamtreaktionsraumes) mit dem identischen Katalysator wie in Vergleichsbeispiel 1 befüllt. In der zweiten, unteren Reaktionszone (40 % des Gesamtreaktionsraumes) befand sich der identische Katalysator wie in Vergleichsbeispiel 2. Bei einer Wandtemperatur von 320 °C, einer Reaktionstemperatur zwischen 320 und 380 °C innerhalb der ersten Reaktionszone sowie zwischen 320 und 325 °C innerhalb der zweiten Reaktionszone stellte sich am Reaktorausgang ein Umsatzgrad an Methanol von 99 % ein. Die Selektivität zu Methylmercaptan betrug 95,5 %, die Ausbeute an Methylmercaptan 94,5 %.

## Patentansprüche

1. Verfahren zur Herstellung von Alkylmercaptanen durch katalytische Gasphasenumsetzung von Alkanolen und Schwefelwasserstoff an einem Alkaliwolframat, wobei die Umsetzung der Reaktanten in mindestens zwei aufeinanderfolgenden Reaktionszonen erfolgt, die unterschiedliche Katalysatoren mit an die Konzentrationsverhältnisse der Reaktanten in diesen Zonen in Hinblick auf die Mercaptanbildung angepassten Aktivitäten enthalten, wobei die Aktivität des in der ersten Reaktionszone eingesetzten Katalysators bezogen auf die Alkylmercaptanbildung niedriger ist als die des in der zweiten oder weiteren Reaktionszone(n) eingesetzten Katalysators.

2. Verfahren gemäß Anspruch 1, bei dem der Katalysator in der zweiten Zone die Umsetzung des entstandenen Alkylmercaptans zu Nebenprodukten mit einer Selektivität von ≤ 1 % katalysiert.

3. Verfahren gemäß Anspruch 1, bei dem der Katalysator in der ersten Zone die Umsetzung von Alkanolen und Schwefelwasserstoff zu Nebenprodukten mit einer Selektivität von ≤ 1 % katalysiert.

4. Verfahren gemäß den Ansprüchen 1 bis 3, bei dem man halogenidhaltige und/oder halogenidfreie Alkaliwolframate als Katalysatoren einsetzt.

5. Verfahren gemäß den Ansprüchen 1 bis 4, bei dem man in der ersten Reaktionszone ein halogenidfreies und in der zweiten Reaktionszone ein halogenidhaltiges Alkaliwolframat einsetzt.

6. Verfahren gemäß Anspruch 5, **dadurch gekennzeichnet, dass** man ein bromidhaltiges Alkaliwolframat einsetzt.

7. Verfahren gemäß den Ansprüchen 1 bis 6, bei dem der Wechsel zu einem Katalysator mit einer gegenüber dem in den ersten Reaktionszone eingesetzten höheren Aktivität für die Mercaptanbildung nach der Umsetzung von 30 bis 95% des eingesetzten Alkohols in der ersten Reaktionszone erfolgt.

8. Verfahren gemäß den Ansprüchen 1 bis 7, bei dem die geringere Aktivität des Katalysators in der ersten Reaktionszone durch die Verdünnung der Katalysatormenge mit festem Inertmaterial erreicht wird.

9. Verfahren gemäß Anspruch 8, bei dem in der ersten und den zweiten oder weiteren Reaktionszone(n) derselbe Katalysator eingesetzt wird.

10. Verfahren gemäß den Ansprüchen 1 bis 7, bei dem die geringere Aktvitität des Katalysators in der ersten Reaktionszone durch die Variation von einer oder mehreren physikalischen Eigenschaften ausgewählt aus der Gruppe spezifische Oberfläche, Porenradienverteilung, Porenvolumen, Pelletdurchmesser und Pelletform erreicht wird.

11. Verfahren gemäß den Ansprüchen 1 bis 10, bei dem die Reaktionszonen in unterschiedlichen Reaktoren, die miteinander in Reihe geschaltet sind, aufeinander folgen.

12. Verfahren gemäß den Ansprüchen 1 bis 10, bei dem die Reaktionszonen direkt miteinander verbunden sind.

13. Verfahren gemäß den Ansprüchen 1 bis 12, bei dem man Schwefelwasserstoff und Methanol zu Methylmercaptan umsetzt.

## Claims

1. Process for preparing alkyl mercaptans by catalytic gas phase reaction of alkanols and hydrogen sulphide over an alkali metal tungstate, the reactants being converted in at least two successive reaction zones which contain different catalysts having activities with respect to mercaptan formation adjusted to the concentration ratios of the reactants in these zones, the activity of the catalyst used in the first reaction zone, based on alkyl mercaptan formation, being lower than that of the catalyst used in the second or further reaction zone(s).

2. Process according to Claim 1, in which the catalyst in the second zone catalyses the conversion of the alkyl mercaptan formed to by-products with a selectivity of ≤ 1%.

3. Process according to Claim 1, in which the catalyst in the first zone catalyses the reaction of alkanols and hydrogen sulphide to by-products with a selectivity of ≤ 1%.

4. Process according to Claims 1 to 3, in which halide containing and/or halide-free alkali metal tungstates are used as catalysts.

5. Process according to Claims 1 to 4, in which a halide-free alkali metal tungstate is used in the first reaction zone and a halide containing alkali metal tungstate in the second reaction zone.

6. Process according to Claim 5, **characterized in that** a bromide containing alkali metal tungstate is used.

7. Process according to Claims 1 to 6, in which the change to a catalyst having a higher activity for mercaptan formation compared to that used in the first reaction zone is effected after the reaction of 30 to 95% of the alcohol used in the first reaction zone.

8. Process according to Claims 1 to 7, in which the lower activity of the catalyst in the first reaction zone is achieved by the dilution of the amount of catalyst with solid inert material.

9. Process according to Claim 8, in which the same catalyst is used in the first and the second or further reaction zone(s).

10. Process according to Claims 1 to 7, in which the lower activity of the catalyst in the first reaction zone is achieved by the variation of one or more physical properties selected from the group of specific surface area, pore radius distribution, pore volume, pellet diameter and pellet form.

11. Process according to Claims 1 to 10, in which the reaction zones follow in succession in different reactors which are connected to one another in series.

12. Process according to Claims 1 to 10, in which the reaction zones are connected to one another directly.

13. Process according to Claims 1 to 12, in which hydrogen sulphide and methanol are reacted to give methyl mercaptan.

## Revendications

1. Procédé pour la préparation d'alkylmercaptans par transformation catalytique en phase gazeuse d'alcanols et d'acide sulfhydrique sur un tungstate de métal alcalin, la transformation des réactifs ayant lieu dans au moins deux zones de réaction successives, qui contiennent des catalyseurs différents présentant des activités, en ce qui concerne la formation de mercaptans, adaptées aux rapports des concentrations des réactifs dans ces zones, l'activité du catalyseur utilisé dans la première zone de réaction, par rapport à la formation d'alkylmercaptans, étant inférieure à celle du catalyseur utilisé dans la deuxième ou les autres zone(s) de réaction.

2. Procédé selon la revendication 1, dans lequel le catalyseur dans la deuxième zone catalyse la transformation de l'alkylmercaptan formé en produits secondaires à une sélectivité ≤ 1%.

3. Procédé selon la revendication 1, dans lequel le catalyseur dans la première zone catalyse la transformation d'alcanols et d'acide sulfhydrique en produits secondaires à une sélectivité ≤ 1%.

4. Procédé selon les revendications 1 à 3, dans lequel on utilise des tungstates de métal alcalin contenant halogénure et/ou exempts d'halogénure comme catalyseurs.

5. Procédé selon les revendications 1 à 4, dans lequel on utilise, dans la première zone de réaction, un tungstate de métal alcalin exempt d'halogénure et, dans la deuxième zone de réaction, un tungstate de métal alcalin contenant halogénure.

6. Procédé selon la revendication 5, **caractérisé en ce qu'**on utilise un tungstate de métal alcalin contenant bromure.

7. Procédé selon les revendications 1 à 6, dans lequel le changement en un catalyseur présentant une activité, pour la formation de mercaptans, supérieure par rapport à celui dans la première zone de réaction a lieu après la transformation de 30 à 95% de l'alcanol utilisé dans la première zone de réaction.

8. Procédé selon les revendications 1 à 7, dans lequel l'activité plus basse du catalyseur dans la première zone de réaction est obtenue par dilution de la quantité de catalyseur par un matériau inerte solide.

9. Procédé selon la revendication 8, dans lequel on utilise le même catalyseur dans la première et la deuxième ou les autres zone(s) de réaction.

10. Procédé selon les revendications 1 à 7, dans lequel l'activité plus basse du catalyseur dans la première zone de réaction est obtenue par variation d'une ou de plusieurs propriétés physiques choisies dans le groupe formé par la surface spécifique, la distribution des rayons des pores, le volume des pores, le diamètre des pellets et la forme des pellets.

11. Procédé selon les revendications 1 à 10, dans lequel les zones de réaction se succèdent dans des réacteurs différents, qui sont commutés en série les uns avec les autres.

12. Procédé selon les revendications 1 à 10, dans lequel les zones de réaction sont reliées directement les unes aux autres.

13. Procédé selon les revendications 1 à 12, dans lequel on transforme de l'acide sulfhydrique et du méthanol en méthylmercaptan.
